# EUROPEAN PATENT APPLICATION

(11) **EP 4 516 808 A1**
(43) Date of publication of application: **05.03.2025**
(21) Application number: 24194553.4
(22) Date of filing: 14.08.2024
(51) Int. Cl.: C07K 16/28

(54) **TARGETED CARRIER WITH A PROPERTY OF CROSSING THE BLOOD-BRAIN BARRIER, PHARMACEUTICAL COMPOSITION AND USE THEREOF**

(30) Priority: 14.08.2023 US 202363519434 P
(71) Applicant: China Medical University, Taichung City 406040 (TW)
(72) Inventor: Cho, Der-Yang, 404 Taichung City (TW); Chiu, Shao-Chih, 404 Taichung City (TW); Chen, Yi-Wen, 404 Taichung City (TW); Shie, Ming-You, 404 Taichung City (TW); Pan, Chih-Ming, 404 Taichung City (TW); Huang, Shi-Wei, 404 Taichung City (TW); Chen, Yen, 404 Taichung City (TW); Chen, Cheng-Yu, 404 Taichung City (TW); Kan, Kai-Wen, 404 Taichung City (TW)
(74) Representative: Cabinet Chaillot

(57) **Abstract**

A drug carrier with a property of crossing the blood-brain barrier comprises an extracellular vesicle with a human leukocyte antigen-G antibody on its surface. This carrier can serve as a pharmaceutical composition for promoting apoptosis of brain tumor cells, inhibiting growth of brain tumor cells, or reducing expression of 06-methylguanine-DNA methyltransferase (MGMT) in brain tumor cells. These effects contribute to the treatment of glioblastoma multiforme (GBM).

## Description

### FIELD OF INVENTION

Drug carrier, in particular a targeted carrier with a property of crossing the blood-brain barrier, and its use.

### BACKGROUND OF THE INVENTION

Glioblastoma multiforme (GBM) is the most common and aggressive malignant tumor of the brain. These tumor cells are notoriously difficult to treat. Traditionally, a standard treatment involves surgical resection followed by a post-operative radiation therapy. However, this approach can only partially eliminate the cancer cells. In addition, GBM often develops resistance to the sole oral chemotherapy drug, Temodal, due to the expression of O6-methylguanine-DNA methyltransferase (MGMT). As a result, patients typically experience an average prolongation of life of only 10 months to one year after treatment, with a survival rate of less than 5%. Consequently, the development of effective treatments for glioblastoma multiforme (GBM) is a critical and urgent focus of current medical research.

### SUMMARY OF THE INVENTION

A targeted carrier with a property of crossing the blood-brain barrier, comprising an extracellular vesicle, which has a human leukocyte antigen-G antibody bound to its surface.

Wherein, the extracellular vesicle is bound to the human leukocyte antigen-G antibody through a transmembrane protein.

Wherein, the transmembrane protein expresses a single-domain antibody fragment that binds to the human leukocyte antigen-G antibody.

Wherein, the single-domain antibody fragment is a variable domain of heavy chain of heavy-chain antibody (VHH) or a single-chain variable fragment (scFv).

Wherein, the transmembrane protein is selected from the group consisting of CD68, CD81, and CD9.

Wherein, the targeted carrier contains a microRNA or a drug.

Furthermore, the present invention also provides a use of the aforementioned targeted carrier including promoting apoptosis of the brain tumor cell, inhibiting the growth of the brain tumor cell, or reducing the expression of O6-methylguanine-DNA methyltransferase (MGMT) in the brain tumor cell.

The targeted carrier provided by the present invention can effectively cross the blood-brain barrier, specifically reach the location of the tumor cells, and release drugs to achieve excellent therapeutic effects in promoting the tumor cells toward apoptosis.

### BRIEF DESCRIPTION OF THE DRAWINGS

FIG. 1A is an analysis diagram of GBM cell lines in a preferred embodiment of the present invention;
FIG. 1B is a fluorescence diagram of GBM cell lines in a preferred embodiment of the present invention;
FIG. 2A is a surface plasmon resonance test diagram of the drug carrier in a preferred embodiment of the present invention;
FIG. 2B is a microscopic imaging diagram of the drug carrier in a preferred embodiment of the present invention;
FIG. 2C is a nanoparticle tracking analysis diagram of the drug carrier in a preferred embodiment of the present invention;
FIG. 2D is a quantitative analysis diagram of transmembrane proteins of the drug carrier in a preferred embodiment of the present invention;
FIG. 2E is an expression analysis diagram of transmembrane proteins of the drug carrier in a preferred embodiment of the present invention;
FIG. 2F is a quantitative analysis diagram of HLA-G antibodies of the drug carrier in a preferred embodiment of the present invention;
FIG. 3 is a fluorescence staining diagram of GBM cell lines in a preferred embodiment of the present invention;
FIG. 4A is a loading capacity analysis diagram of the drug carrier in a preferred embodiment of the present invention;
FIG. 4B is an endocytosis analysis diagram of GBM cell lines in a preferred embodiment of the present invention;
FIG. 5 is a protein expression analysis diagram of GBM cell lines in a preferred embodiment of the present invention;
FIG. 6 is a schematic diagram of a blood-brain barrier cell culture model in a preferred embodiment of the present invention;
FIG. 7 is a death rate trend diagram of GBM cell lines in a preferred embodiment of the present invention;
FIG. 8A is a fluorescence staining diagram of BAX protein expression in GBM cell lines in a preferred embodiment of the present invention;
FIG. 8B is an apoptotic factor analysis diagram of GBM cell lines in a preferred embodiment of the present invention;
FIG. 9 is a MGMT protein expression analysis diagram of cells in a preferred embodiment of the present invention;
FIG. 10A is an image of drug carrier distribution in an animal model in a preferred embodiment of the present invention;
FIG. 10B is a mouse body weight trend diagram in an animal model in a preferred embodiment of the present invention;
FIG. 10C is a survival rate trend diagram in an animal model in a preferred embodiment of the present invention;
FIG. 10D is a growth trend diagram of GBM cell lines in an animal model in a preferred embodiment of the present invention;
FIG. 10E is a tissue section staining diagram in an animal model in a preferred embodiment of the present invention;
FIG. 11A is a fluorescence diagram of the first specimen in a preferred embodiment of the present invention;
FIG. 11B is a fluorescence diagram of the drug carrier expression in the first specimen in a preferred embodiment of the present invention;
FIG. 11C is a MGMT protein expression analysis diagram of the first specimen in a preferred embodiment of the present invention; and
FIG. 11D is a cell death rate trend diagram of the first specimen in a preferred embodiment of the present invention.

### DETAILED DESCRIPTION OF THE PREFERRED EMBODIMENTS

It should be understood that the term "extracellular vesicles (EV)" as used herein refers to bilayer lipid vesicles secreted by cells into the extracellular space. The extracellular vesicles include exosomes and microvesicles, which serve as a new form of intercellular communication and are a new field of research in physiology, pathology, nanoscience, and other fields in recent years. The extracellular vesicles are highly heterogeneous vesicular bodies secreted by cells, containing proteins, mRNA/miRNA, DNA, lipids, etc. They are classified according to their biogenesis, release pathways, size, content, and function, and are mainly purified based on particle size.

It should be understood that the following description combines the specification, drawings, and specific embodiments to explain the present invention, but the embodiments do not limit the present invention in any form. Unless otherwise indicated, the reagents, methods, and apparatus used in the present invention are conventional reagents, methods, and apparatus in the art.

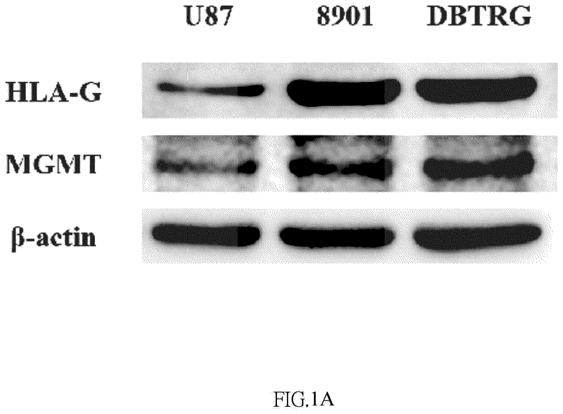

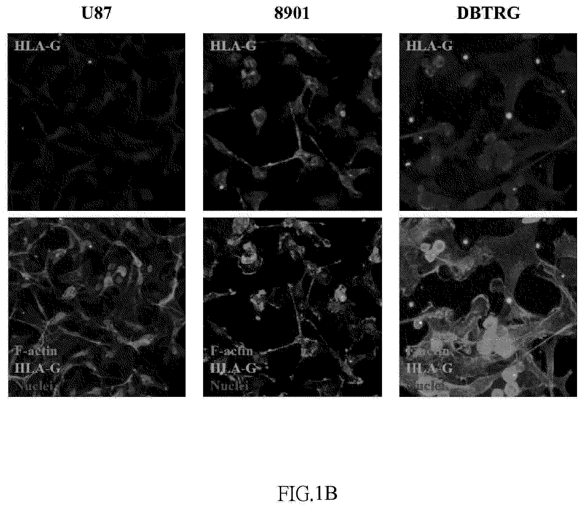

Referring to FIGS. 1A and 1B, the present invention first uses Western blotting and fluorescence staining techniques to demonstrate the high expression of O6-methylguanine-DNA methyltransferase (MGMT) protein in three different glioblastoma multiforme (GBM) cell lines (U87 cell line, 8901 cell line, and DBTRG cell line). Human leukocyte antigen G (HLA-G) is an important immunomodulatory molecule. Many studies have shown that the HLA-G is abnormally highly expressed on the surface of various tumor cellswhich may inhibit the immune response of tumor cells and prevent tumor cells from being attacked by the immune system.

In the Western blotting results of FIG. 1A, the expression of MGMT and HLA-G can be seen in U87, 8901, and DBTRG cells. In the fluorescence staining of FIG. 1B, F-actin is labeled with red fluorescence to define the cell area; nuclei are labeled with blue fluorescence to define the location of cell nuclei; and HLA-G protein is labeled with green fluorescence to confirm the expression of HLA-G. It can be seen that the HLA-G protein is expressed within the cells.

The present invention provides a targeted drug carrier (αHLA-G EV), comprising an extracellular vesicle with a human leukocyte antigen-G antibody (HLA-G antibody) bound on the surface of extracellular vesicle. The drug carrier (αHLA-G EV) can achieve specific targeted binding with the HLA-G protein of GBM tumor cells through the HLA-G antibody and promote endocytosis of GBM tumor cells, while avoiding the binding reaction between normal cells and the drug carrier (αHLA-G EV).

The extracellular vesicle is a bilayer lipid vesicle secreted by a source cell. After culturing the cells, the culture medium is collected and the extracellular vesicle can be extracted from the culture medium through conventional separation techniques such as centrifugation and filtration.

Preferably, the source cell can be human embryonic kidney cells 293 (HEK293) cell line or its derived cell lines, such as HEK293T, HEK293F, HEK293A, or HEK293E.

Preferably, the source cell can be mesenchymal stem cells (MSC).

The extracellular vesicle can bind to the HLA-G antibody through a transmembrane protein. The transmembrane protein can be CD63, CD81, or CD9.

The sequence of the HLA-G antibody can be inserted into the the source cell to produce an extracellular vesicle that expresses the HLA-G antibody.

Preferably, the transmembrane protein of the extracellular vesicle expresses a single-domain antibody fragment, allowing the single-domain antibody fragment to serve as a specific binding site between the transmembrane protein and the HLA-G antibody.

The single-domain antibody fragment is a variable domain of heavy chain of heavy-chain antibody (VHH) or a single-chain variable fragment (scFv). Furthermore, the expression of the single-domain antibody fragment by the transmembrane protein can be achieved through genetic transformation, genetic engineering, and other techniques.

Furthermore, in this embodiment, molecular cloning techniques are used to synthesize a synthetic sequence concatenating the HLA-G antibody/single-domain antibody fragment/transmembrane protein, and the synthetic sequence is inserted into a pcDNA3.4 plasmid (Thermo Scientific) to create an HLA-G antibody expression plasmid. Then, using Lipofectamine 3000 reagent (Thermo Scientific), the HLA-G antibody expression plasmid is transfected into the source cells for culture, and the extracellular vesicles secreted by the source cells expressing the HLA-G antibody are collected.

The present invention will subsequently use the transmembrane protein CD63 expressing the single-domain antibody fragment bound to the HLA-G antibody as an embodiment for explanation, and will use an ordinary extracellular vesicle (EV) without binding to the HLA-G protein antibody as a comparative example.

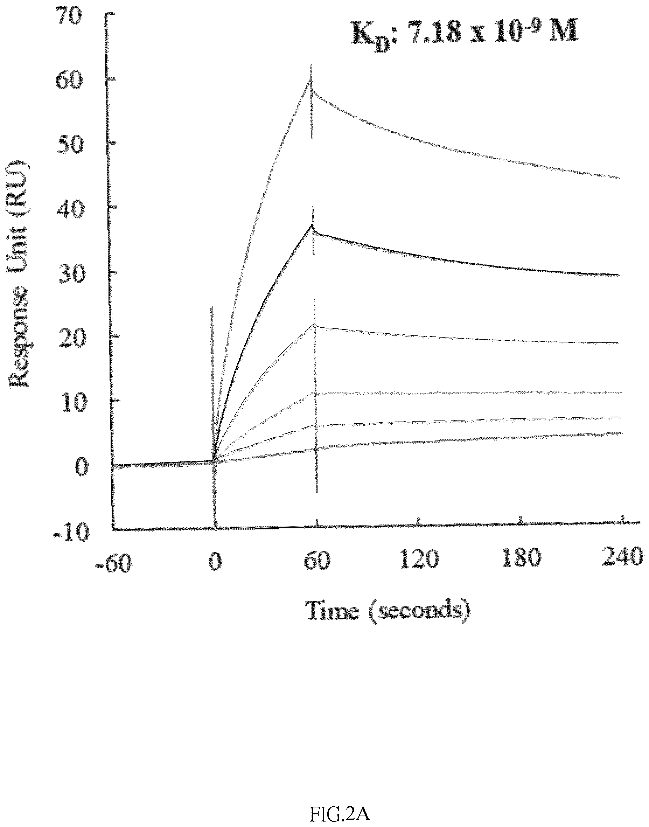

Referring to FIG. 2A, surface plasmon resonance (SPR) technology is used to detect the reflection signal (response unit) of the drug carrier at different concentrations to obtain an affinity parameter (affinity, K_{D} value) of the drug carrier (αHLA-G EV). In this embodiment, the concentration range of the drug carrier used for detection by surface plasmon resonance technology is between 25 nM and 0.78125 nM. After detection and numerical conversion, it can be seen that the affinity parameter of the drug carrier is 7.18 x 10⁻⁹ of the K_{D} value (molar concentration, M). The K_{D} value below 10⁻⁹ indicates high antibody affinity.

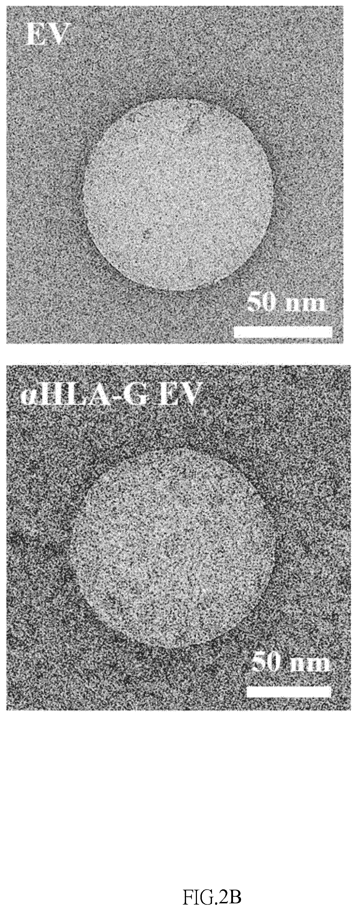

Referring to FIG. 2B, using transmission electron microscope (TEM), it is observed that the particle size difference between the ordinary extracellular vesicle (EV) and the drug carrier (αHLA-G EV) is not significant, confirming that their characteristics are similar.

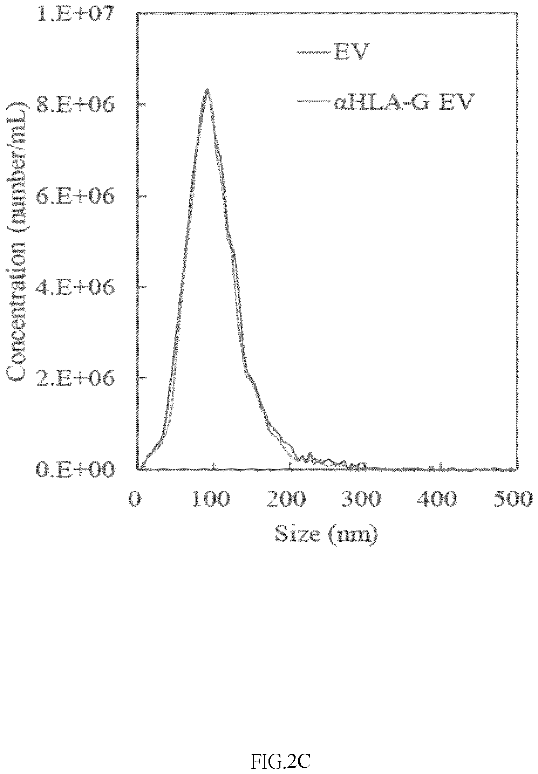

Referring to FIG. 2C, nanoparticle tracking analysis (NTA) is used not only to confirm whether the particle sizes of the ordinary extracellular vesicle (EV) and the drug carrier (αHLA-G EV) are comparable, but also to further detect whether the concentrations of the ordinary extracellular vesicle (EV) and the drug carrier (αHLA-G EV) after extraction are similar. This proves that both the ordinary extracellular vesicle (EV) and the drug carrier (αHLA-G EV) are extracellular vesicles.

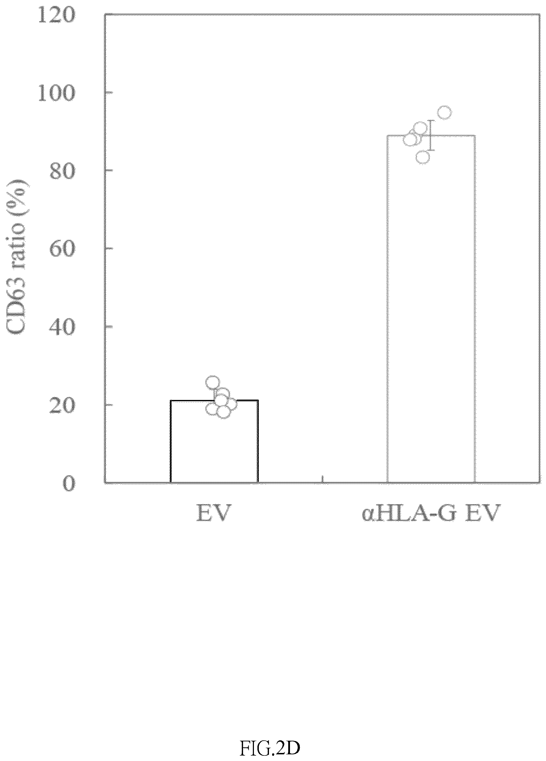

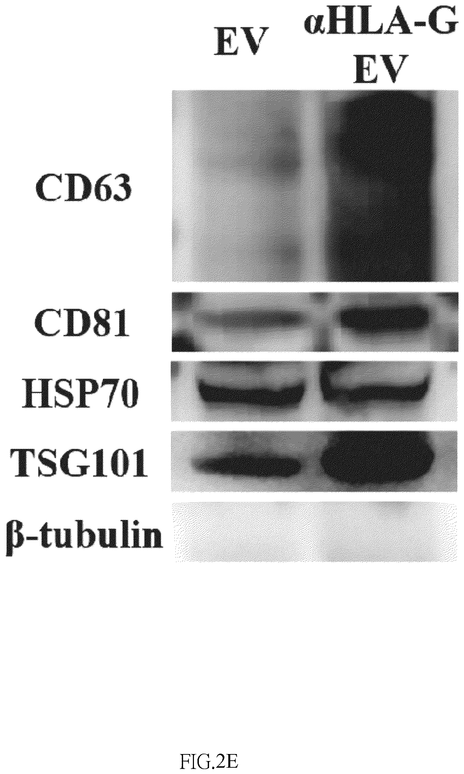

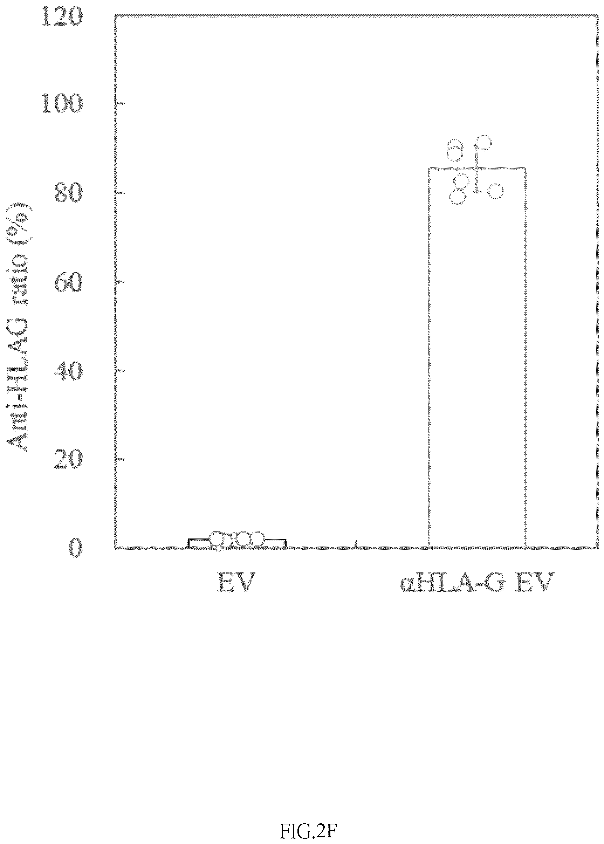

FIGS. 2D and 2E further analyze the proportion of total expression of the transmembrane protein CD63 in the ordinary extracellular vesicle (EV) and the drug carrier (αHLA-G EV) after extraction. It can be seen that the drug carrier (αHLA-G EV) carrying the HLA-G antibody has a total expression rate of transmembrane protein CD63 as high as 90%. Similarly, in the Western blotting results, the expression levels of the transmembrane proteins CD63 and CD81 in the drug carrier (αHLA-G EV) are significantly higher than those in the ordinary extracellular vesicle (EV). FIG. 2F further confirms that the drug carrier (αHLA-G EV) carries the HLA-G antibody.

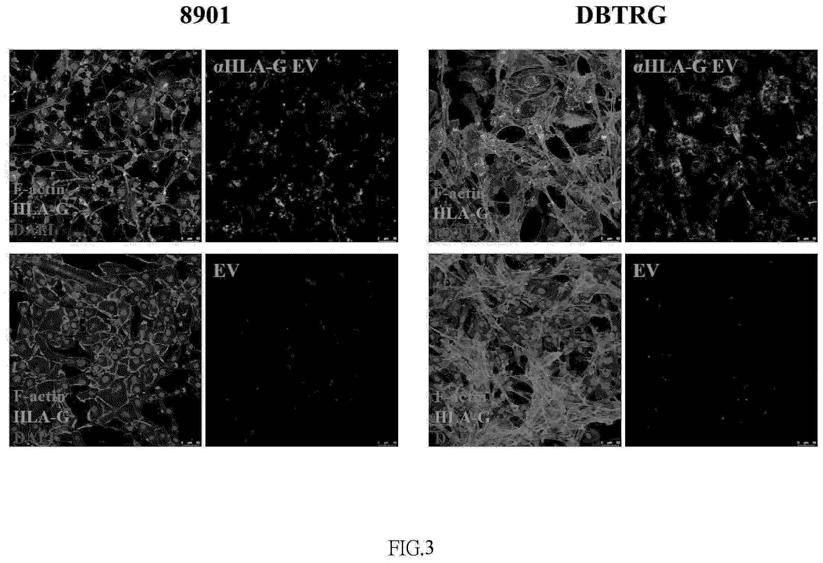

FIG. 3 shows the endocytosis effect of testing the drug carrier (αHLA-G EV) on the glioblastoma multiforme (GBM) cell lines 8901 and the DBTRG, respectively. Red fluorescence labels F-actin to define the cell area; green fluorescence labels extracellular vesicles to confirm whether the extracellular vesicles have entered the cell area. The fluorescence staining results show that the expression of the drug carrier in the GBM cell lines is significantly increased, proving that the drug carrier enhances the affinity with the GBM cell lines through carrying the HLA-G antibody.

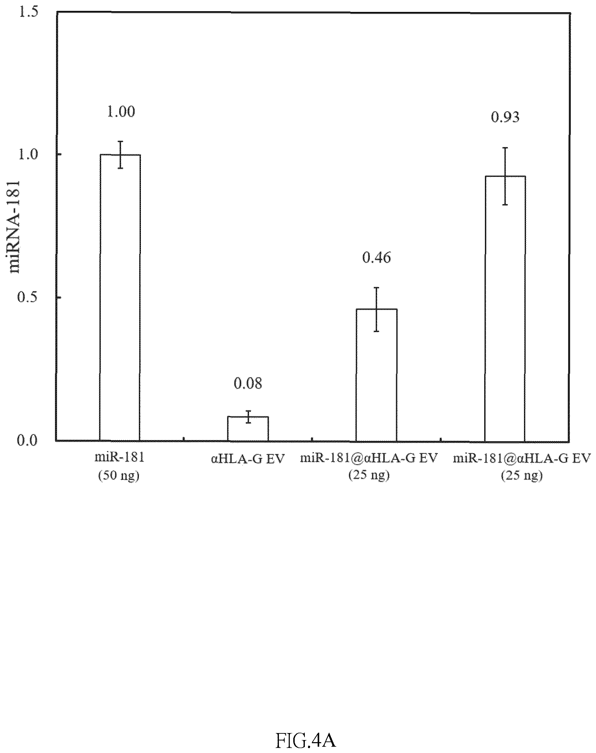

To prove that the drug carrier (αHLA-G EV) can exhibit drug releasing ability after entering cells through endocytosis, the present invention further loads a microRNA-181 (miR-181), which inhibits the MGMT expression, into the drug carrier to ensure that the drug carrier can exhibit MGMT inhibiting effect after entering the GBM cell lines. In FIG. 4A, reverse transcription-PCR (RT-PCR) confirms the expression level of miR-181 in the drug carrier after encapsulation. In this experiment, the expression level of 50ng miR-181 confirmed by RT-PCR is set as the standard value of 1, and comparisons are made between the drug carrier (αHLA-G EV) without loading miR-181, the drug carrier loading 25ng miR-181 (miR-181@αHLA-G EV(25g)), and the drug carrier loading 50ng miR-181 (miR-181@αHLA-G EV(50g)). In the group of the drug carrier loading 50ng miR-181 (miR-181@αHLA-G EV(50g)), it can be demonstrated that the expression in the drug carrier is equivalent to that of the same amount of miR-181 (50ng) when not encapsulated, proving the effectiveness of the drug carrier in loading small molecules (such as miR-181, proteins, or drugs) after binding with the HLA-G antibody.

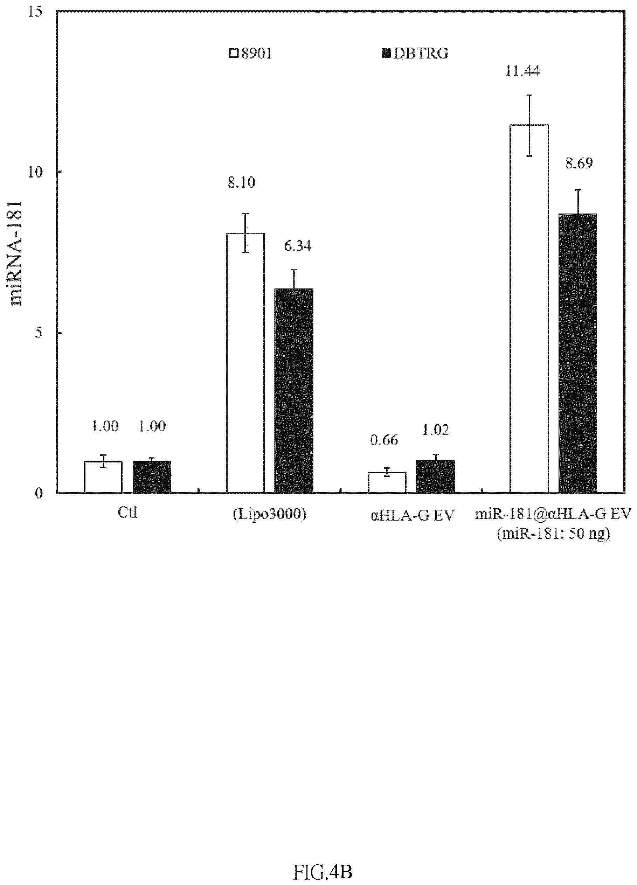

In FIG. 4B, RT-PCR is used to confirm the expression level of miR-181 in each GBM cell line after the GBM cell lines 8901 and DBTRG take up the drug carrier through endocytosis. The unreacted group is used as a first control group (Clt) and set as the standard value of 1; the group in which the commonly used drug carrier Lipo3000 in current technology is loaded with miR-181 and reacts with the GBM cell lines is used as a second control group (Lipo3000); and the group in which the drug carrier reacts with the GBM cell lines is used as a third control group (αHLA-G EV). The results show that in the experimental group (miR-181@αHLA-G EV) where the GBM cell lines are treated with the drug carrier loaded with miR-181, miR-181 has a high expression level in the GBM cell lines, even higher than the commonly used drug carrier Lipo3000 in current technology. This not only proves the loading efficiency of the drug carrier, but also confirms its release capability.

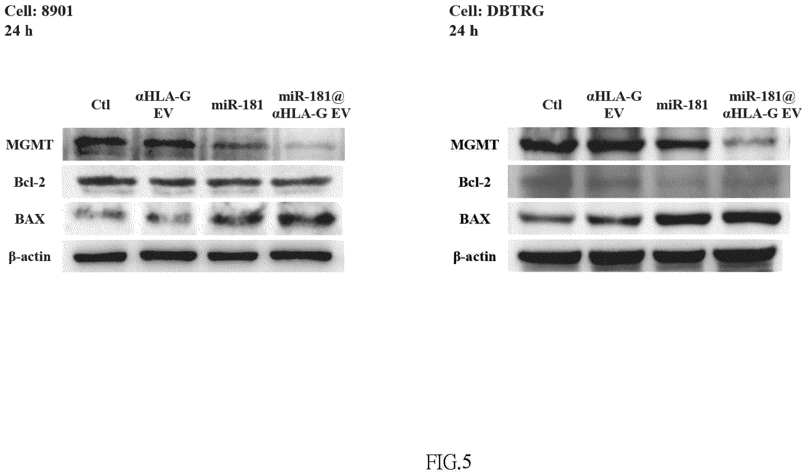

In FIG. 5, the unreacted group is used as a first control group (Clt); the group in which the drug carrier reacts with the GBM is used as a second control group (αHLA-G EV); the group in which the miRNA directly reacts is used as a third control group (miR-181); and the group in which the drug carrier is loaded with the miRNA is used as an experimental group (miR-181@αHLA-G EV). After culturing the above groups with each GBM cell line for 24 hours, Western blotting is used to confirm the protein expression in each GBM cell line of each group. The results show that in the experimental group, the expression of MGMT protein, which is an indicator of resistance to the chemotherapy drug Temodal (TMZ), is significantly decreased in each GBM cell line, demonstrating the excellent downregulating effect of miR-181. Notably, the expression of Bcl-2 protein, an indicator of apoptosis inhibition, is also significantly decreased, while the expression of BAX protein, an indicator of apoptosis promotion, is significantly increased, indicating that after miR-181 reaction, it also helps the GBM cell lines to develop towards the trend of apoptosis.

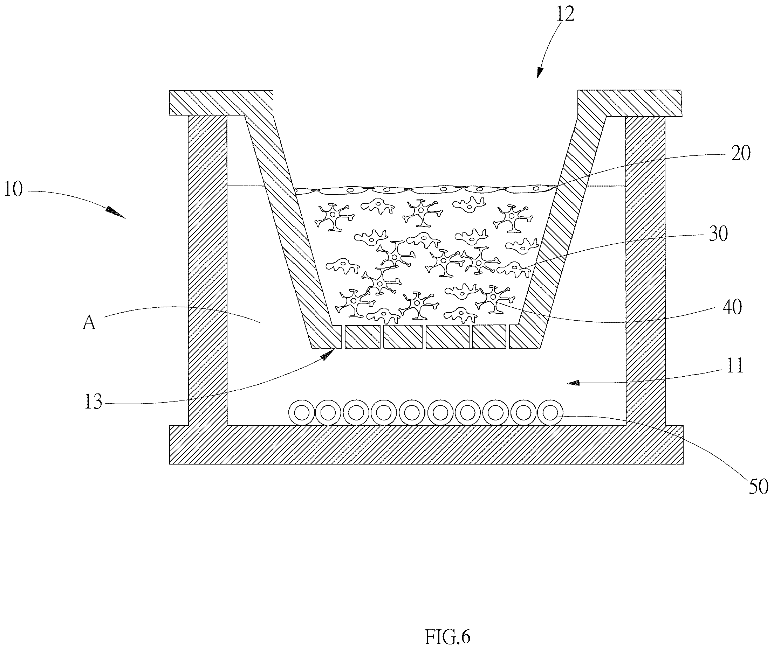

To prove that the drug carrier can be effectively used in living organisms, a blood-brain barrier cell culture model 10 is provided in FIG. 6, which is intended to simulate the blood-brain barrier environment in living organisms to ensure that the drug carrier can cross the blood-brain barrier to enter the brain region and specifically bind to the GBM cells. The blood-brain barrier cell culture model 10 includes a first culture area 11 and a second culture area 12, with multiple perforations 13 connecting the first culture area 11 and the second culture area 12. The first culture area 11 and the second culture area 12 are filled with a hydrogel A. The GBM cell line U87 is cultured in the hydrogel A of the first culture area 11, while endothelial cells 20, pericytes 30, and astrocytes 40 are sequentially arranged in the second culture area 12. Wherein the resistance value of the blood-brain barrier cell culture model 10 is set to 2144Ω.cm² to establish an impedance environment of the blood-brain barrier. In subsequent experiments, the drug carrier is given to the second culture area 12 of the blood-brain barrier cell culture model 10, and experimental analysis is performed on the GBM cell line U87 in the first culture area 11 to confirm the efficacy and therapeutic effect of the drug carrier crossing from the second culture area 12 to the first culture area 11.

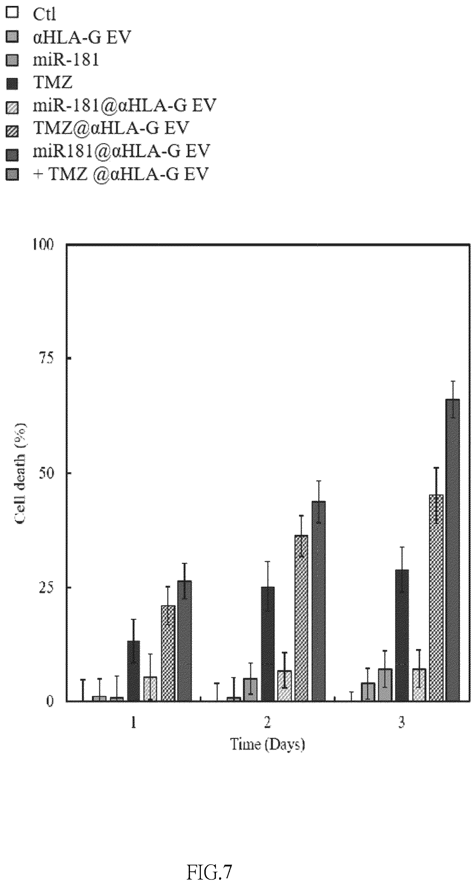

In this experiment, the unreacted group is used as a first control group (Clt); the drug carrier with the HLA-G antibody bound to the surface is used as a second control group (αHLA-G EV); the group in which the miRNA directly reacts is used as a third control group (miR-181); a chemotherapy drug Temodal (TMZ) directly reacting is used as a fourth control group (TMZ); the drug carrier loaded with the miRNA is used as a first experimental group (miR-181@αHLA-G EV); the drug carrier loaded with the chemotherapy drug is used as a second experimental group (TMZ@αHLA-G EV); and the drug carrier loaded with the miRNA and the drug carrier loaded with the chemotherapy drug for simultaneous reaction in a 1:1 ratio is used as a third experimental group (miR-181@αHLA-G EV + TMZ@αHLA-G EV). The above groups are tested in the blood-brain barrier cell culture model 10, and the cell death rate (%) of the GBM cells treated by each group is confirmed on the 1st, 2nd, and 3rd day. The results in FIG. 7 show that the GBM cells have a significant cell death rate only in the groups treated with the chemotherapy drug Temodal (TMZ) (as shown in the fourth control group (TMZ), the second experimental group (TMZ@αHLA-G EV), and the third experimental group (miR-181@αHLA-G EV + TMZ@αHLA-G EV)), while in the groups receiving only miR-181, the expression of cell death is limited (as shown in the third control group (miR-181) and the first experimental group (miR-181@αHLA-G EV)).

It is noteworthy that although both the fourth control group and the second experimental group treated with the chemotherapy drug Temodal (TMZ) successfully induced cell death, the expression of cell death tended to moderate by the third day, and there was a tendency to develop drug resistance. However, in the third experimental group (miR-181@αHLA-G EV + TMZ@αHLA-G EV) treated simultaneously with Temodal (TMZ) and miR-181-loaded drug carriers, not only was there a significant toxic effect on the first day, but the cell death rate (%) of the GBM cells also increased significantly by the third day.

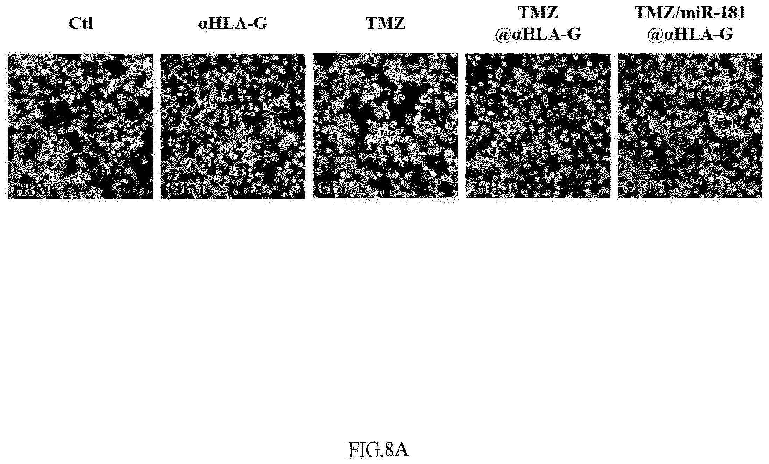

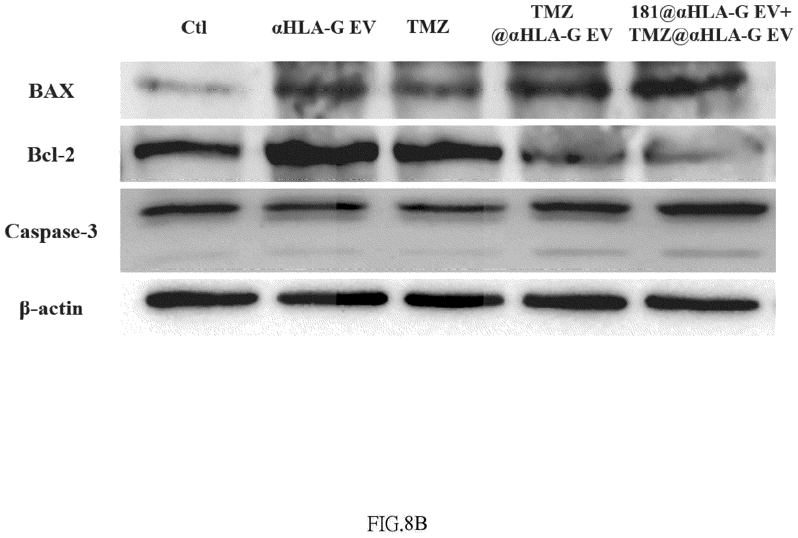

Further analysis of protein expression in the GBM cell line from the aforementioned first control group (Clt), second control group (αHLA-G EV), fourth control group (TMZ), second experimental group (TMZ@αHLA-G EV), and third experimental group (miR-181@αHLA-G EV + TMZ@αHLA-G EV) was conducted using Western blotting and fluorescence staining techniques. In the fluorescence staining of FIG. 8A, the BAX protein was labeled with red fluorescence, while green fluorescence was used to define the cell areas of GBM cells. Similarly, the results showed that HLA-G protein fluorescence was clearly observed only in the GBM cells treated with the chemotherapy drug Temodal (TMZ), and the proportion of BAX protein expression in the GBM cell line of the third experimental group treated with both miRNA-181 and TMZ was significantly higher than that of the fourth control group and the second experimental group. FIG. 8B not only confirmed the above results, but also showed that the expression of the anti-apoptotic indicator Bcl-2 protein was also significantly decreased in the third experimental group, confirming that the GBM cell line in the third experimental group was progressing toward apoptosis.

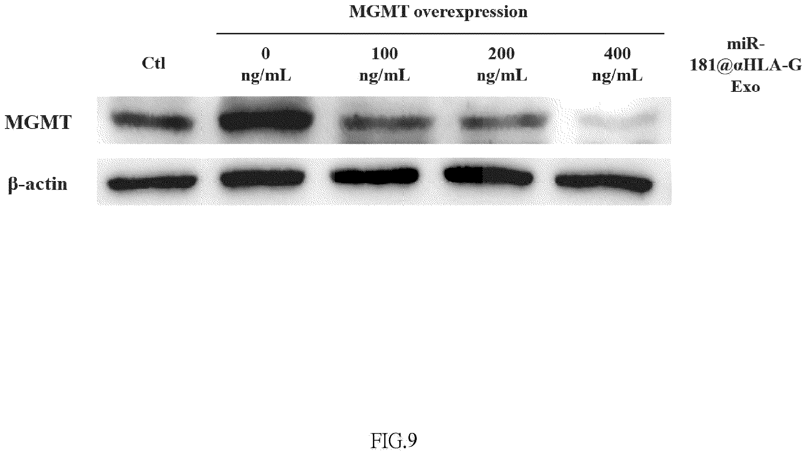

FIG. 9 shows the reaction of drug carriers loaded with miR181 at concentrations of 100 ng/ml, 200 ng/ml and 400 ng/ml with cells overexpressing MGMT protein, demonstrating that the drug carrier can effectively overcome the blood-brain barrier cell culture model 10 and promote the miR181 to downregulate the MGMT gene and protein expression. The results confirm the previous experiments and show that as the concentration of miR181 loaded in the drug carrier increases, the level of the MGMT protein in cells overexpressing MGMT protein decreases accordingly.

In summary, the above experimental results demonstrate that the drug carrier can effectively overcome the blood-brain barrier environment simulated by the blood-brain barrier cell culture model 10, and promote the release of the miR181 and the anticancer drug, thereby enhancing the expression of apoptotic factors in the GBM cell lines, reducing the drug resistance response of the GBM cell lines, and improving the toxic effect of the anticancer drug.

To corroborate the actual effect of the drug carrier in living organisms, in a further experiment, 10⁶ cells of the GBM cell line (U87) were implanted into the brains of mice to establish an animal model of glioblastoma multiforme. Seven days after the implantation surgery, the mice were administered the chemotherapy drug and/or drug carrier via intravenous injection, and imaging was used to confirm the growth status of the chemotherapy drug and/or drug carrier and the GBM cell line in the mouse brain.

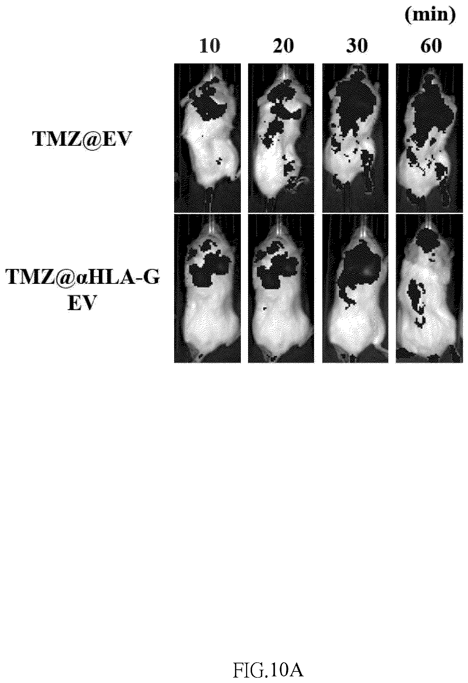

In FIG. 10A, the anticancer drug loaded in ordinary extracellular vesicles was used as a control group (TMZ@ EV), and the anticancer drug loaded in the drug carrier was used as an experimental group (TMZ@αHLA-G EV). At 60 minutes after intravenous injection, it can be observed that the ordinary extracellular vesicles in the control group were widely distributed in the upper body region of the experimental mice (especially in the lung area), while the drug carrier in the experimental group (TMZ@αHLA-G EV) was clearly concentrated in the brain area of the experimental mice, demonstrating that the drug carrier (αHLA-G EV) possesses targeting specificity.

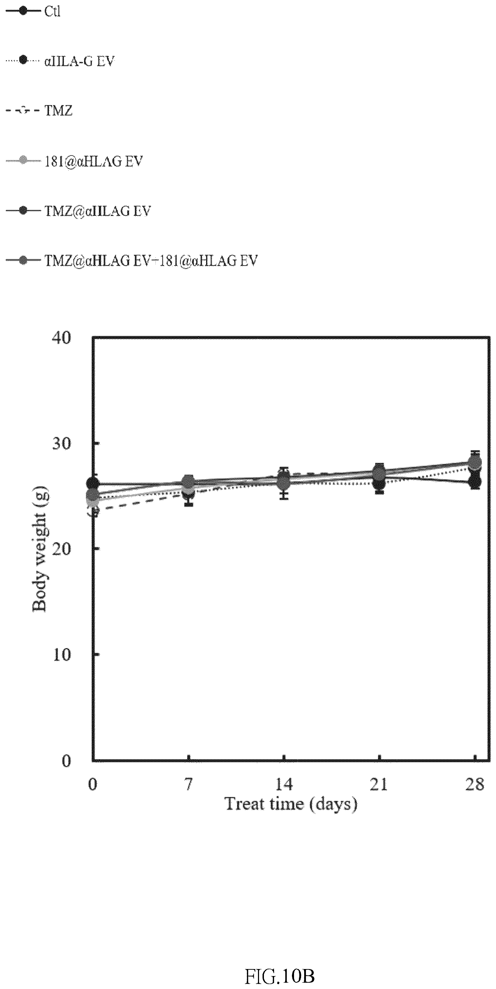

FIGS. 10B to 10D show the animal model, with groups respectively receiving injection of saline as a first control group (Clt), injection of the drug carrier with surface-bound HLA-G antibody as a second control group (αHLA-G EV), injection of the chemotherapy drug Temodal (TMZ) as a third control group (TMZ), injection of miRNA-loaded drug carrier as a first experimental group (miR-181@αHLA-G EV), injection of chemotherapy drug-loaded drug carrier as a second experimental group (TMZ@αHLA-G EV), and injection of a 1:1 mixture of miRNA-loaded and chemotherapy drug-loaded drug carriers as a third experimental group (miR-181@αHLA-G EV + TMZ@αHLA-G EV). The body weight, survival rate, and growth status of the GBM cell line in the experimental mice of each group were confirmed by imaging every 7 days.

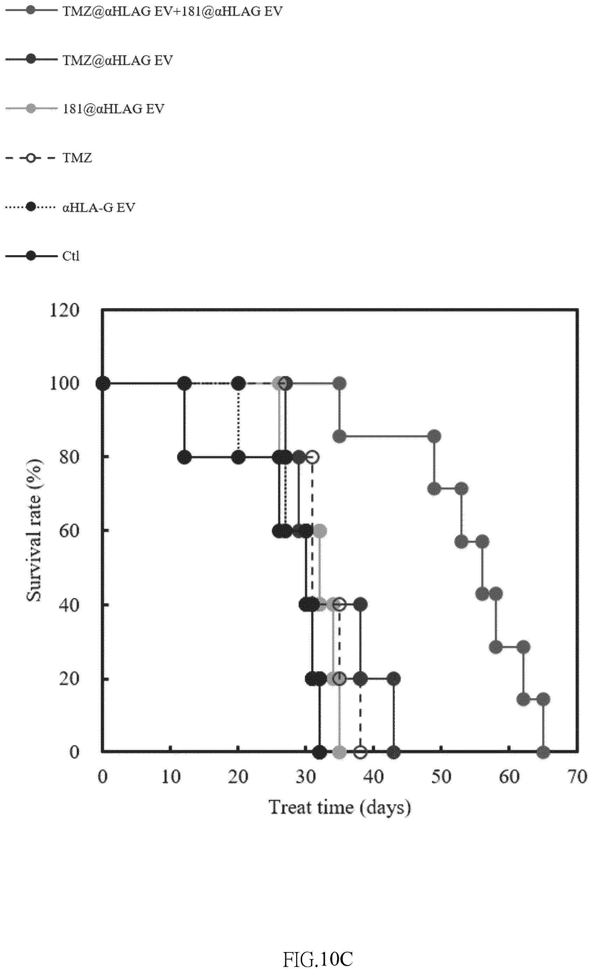

FIG. 10B shows that there are no significant differences in body weight among the experimental mice in each group and no severe changes in physiological conditions due to different injections. However, FIG. 10C shows that the mice in the first control group (Clt), the second control group (αHLA-G EV), and the first experimental group (miR-181@αHLA-G EV) all died within approximately 30 days after injection. The survival rate of the experimental mice increased only in the groups treated with the chemotherapy drug Temodal (TMZ) (such as the third control group and the second experimental group). Notably, in the third experimental group, which was co-treated with the chemotherapy drug Temodal (TMZ) and the miR-181 (miR-181@αHLA-G EV + TMZ@αHLA-G EV), the survival rate of the experimental mice was significantly increased, even to more than 60 days.

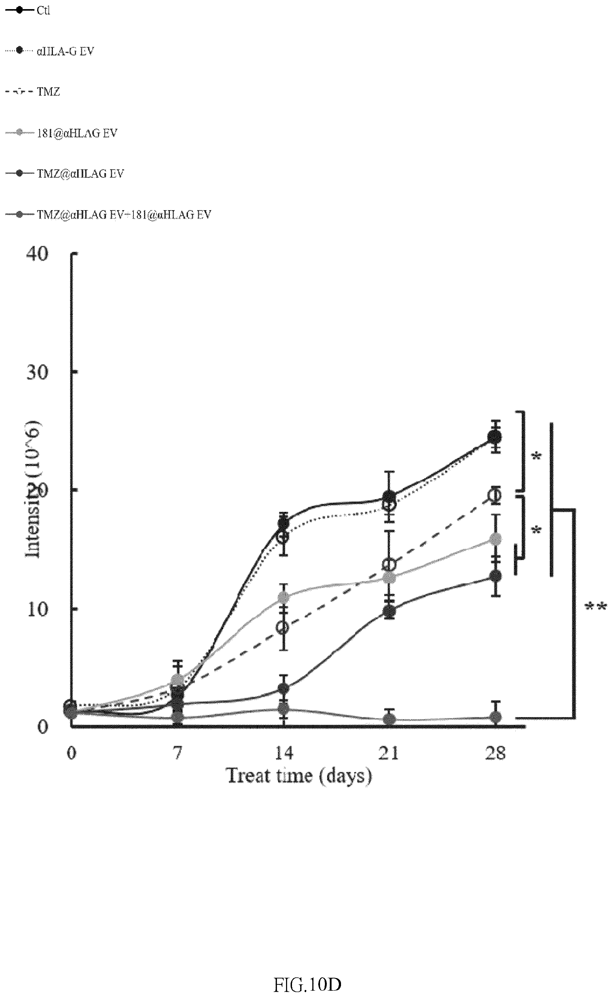

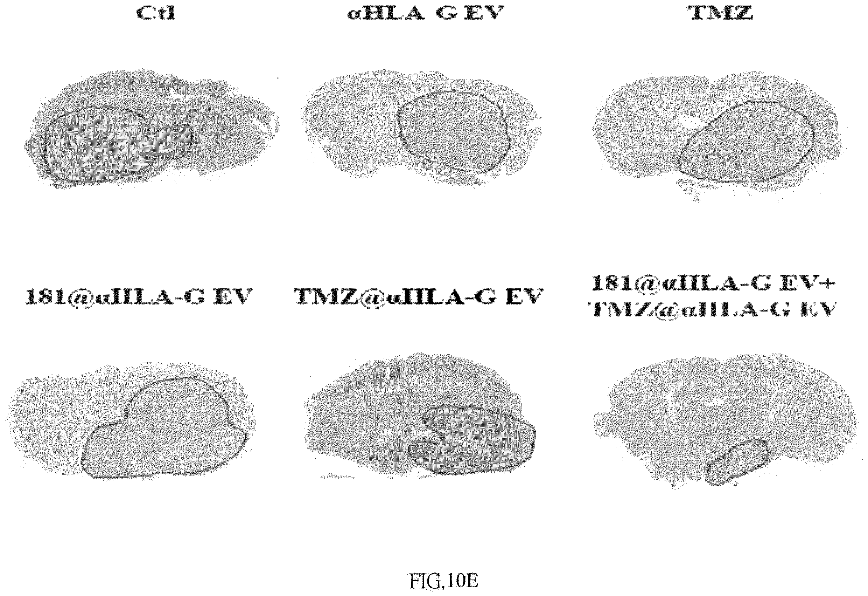

FIGS. 10D and 10E further analyze the fluorescence expression (intensity (10⁶)) of the GBM cell line in the brain area of the experimental mice in each group, and present the tissue morphology through hematoxylin-eosin staining (H&E staining) technique in the tissue section staining images to analyze the growth status of the GBM cells. Compared with the first control group (Clt) and the second control group (αHLA-G EV), the growth of the GBM cell line in the third control group, the first experimental group (miR-181@αHLA-G EV), and the second experimental group (TMZ@αHLA-G EV) showed a gradual increase but was still inhibited to some extent. However, in the third experimental group (miR-181@αHLA-G EV + TMZ@αHLA-G EV), the overall fluorescence response of GBM cells tended to flatten and remain constant, and even showed a decreasing trend on day 21. The tissue section staining images also showed that the distribution area of GBM cells in the third experimental group was significantly smaller than that in the other groups. This confirms that the simultaneous administration of the miR-181 and TMZ-loaded drug carrier to the experimental mice can produce a significant synergistic effect in inhibiting the growth of the GBM cell line.

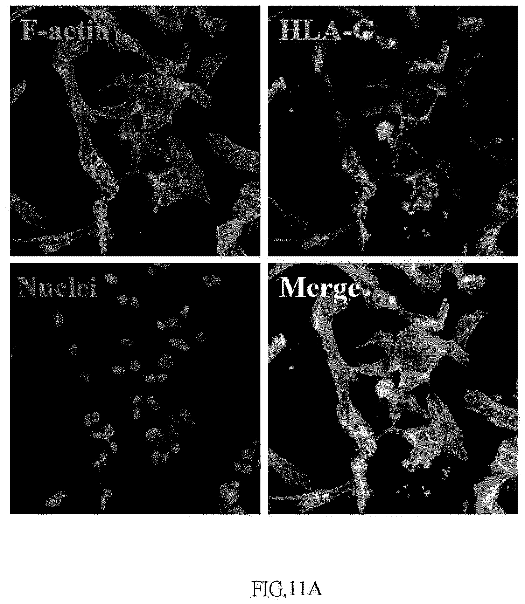

Furthermore, to demonstrate that the drug carrier can produce equally excellent effects on human cells, FIGS. 11A to 11D provide further confirmation using tumor cells from a brain tumor patient. In this case, the brain tumor patient had developed resistance to the anticancer drug TMZ after treatment. As shown in the fluorescence staining in FIG. 11A, F-actin was labeled with red fluorescence to define cell areas; cell nuclei were labeled with blue fluorescence to define nuclear positions; and HLA-G protein was labeled with green fluorescence, allowing observation of the abundant expression of HLA-G protein in the tumor cells.

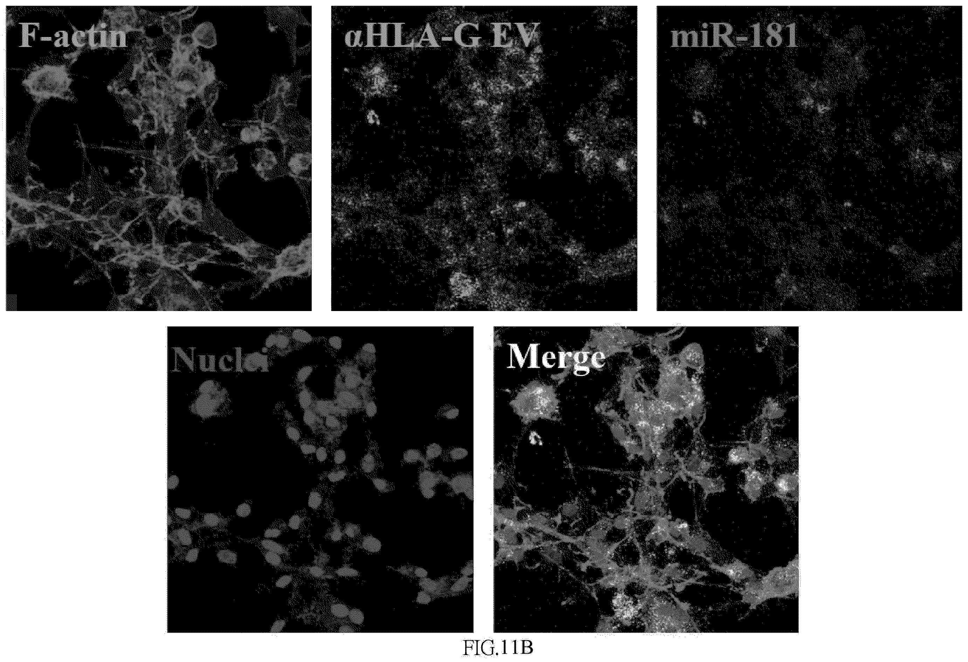

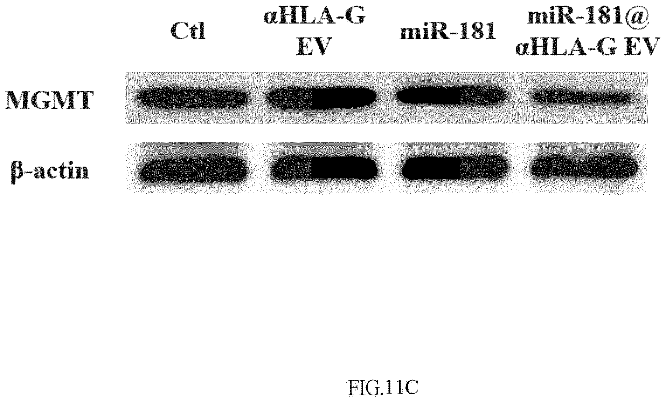

Subsequently, the tumor cells were cultured and treated with the drug carrier loaded with the miR181. In the fluorescence staining of FIG. 11B, F-actin was labeled with purple fluorescence to define cell areas; cell nuclei were labeled with blue fluorescence to define nuclear positions; the drug carrier was labeled with green fluorescence; and the miR181 was labeled with red fluorescence. This demonstrates that the tumor cells internalized the drug carrier and the miR181 through endocytosis, resulting in their expression within the cells. FIG. 11C also confirms that after treatment with the miR181-loaded drug carrier (miR-181@αHLA-G EV), the expression of the MGMT in the tumor cells was significantly reduced compared to the groups treated with only the drug carrier (αHLA-G EV) or the miR181 alone. Additionally, the expression of the anti-apoptotic factor Bcl-2 also showed a downward trend, while the pro-apoptotic factor BAX showed a significant upward trend in expression.

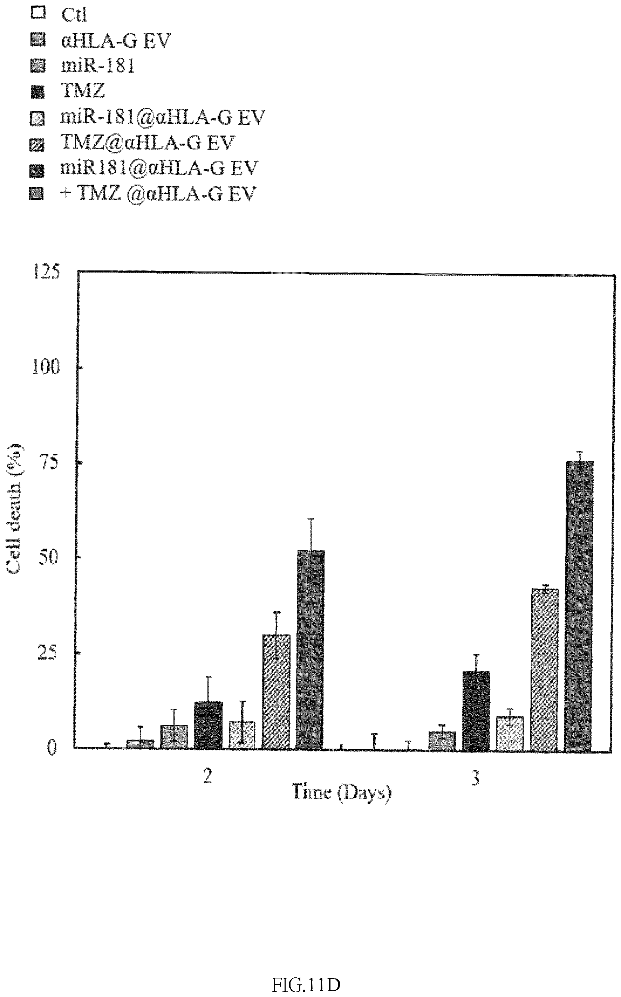

FIG. 11D compares the cell death rates of the tumor cells under different treatment conditions. In this experiment, the groups were also set up as follows: the untreated group as a first control group (Clt); the drug carrier with the surface-bound HLA-G antibody as a second control group (αHLA-G EV); direct treatment with the miRNA as a third control group (miR-181); direct treatment with the chemotherapy drug Temodal (TMZ) as a fourth control group (TMZ); miRNA-loaded drug carrier as a first experimental group (miR-181@αHLA-G EV); chemotherapy drug-loaded drug carrier as a second experimental group (TMZ@αHLA-G EV); and simultaneous treatment with a 1:1 ratio of the miRNA-loaded drug carrier and the chemotherapy drug-loaded drug carrier as a third experimental group (miR-181@αHLA-G EV + TMZ@αHLA-G EV). As in the previous paragraphs, the third experimental group demonstrated excellent effects in promoting tumor cell apoptosis.

The drug carrier provided by the present invention can effectively cross the blood-brain barrier, specifically reach the location of tumor cells, and release drugs to achieve excellent therapeutic effects in promoting tumor cell apoptosis.

## Claims

1. A targeted carrier with a property of crossing the blood-brain barrier, comprising an extracellular vesicle, which comprises a human leukocyte antigen-G antibody on its surface.

2. The targeted carrier according to Claim 1, wherein the extracellular vesicle is bound to the human leukocyte antigen-G antibody through a transmembrane protein.

3. The targeted carrier according to Claim 2, wherein the transmembrane protein comprises a single-domain antibody fragment bound to the human leukocyte antigen-G antibody.

4. The targeted carrier according to Claim 2, wherein the transmembrane protein is selected from the group consisting of CD68, CD81, and CD9.

5. The targeted carrier according to Claim 3, wherein the single-domain antibody fragment is a variable domain of heavy chain of heavy-chain antibody (VHH) or a single-chain variable fragment (scFv).

6. The targeted carrier according to Claim 1 to 5, wherein the drug carrier contains a microRNA or a drug.

7. The targeted carrier according to Claim 6, wherein the microRNA includes miRNA-181; and the drug includes Temodal.

8. The targeted carrier with the property of crossing the blood-brain barrier according to Claim 1 for use in reducing the expression of O6-methylguanine-DNA methyltransferase (MGMT) in a brain tumor cell, wherein the drug carrier contains a microRNA, which is miRNA-181.

9. The targeted carrier for use according to Claim 8, wherein the brain tumor cell is glioblastoma multiforme (GBM).

10. The targeted carrier with the property of crossing the blood-brain barrier according to Claim 1 for use in promoting apoptosis of a brain tumor cell, wherein the targeted carrier contains a microRNA or a drug.

11. The targeted carrier for use according to Claim 10, wherein the microRNA includes miRNA-181, and the drug includes Temodal.

12. The targeted carrier for use according to Claim 10 to 11, wherein the expression of Bcl-2 protein decreases and the expression of BAX protein increases in the brain tumor cell.

13. The targeted carrier for use according to Claim 10 to 11, wherein the brain tumor cell is glioblastoma multiforme (GBM).

14. The targeted carrier with the property of crossing the blood-brain barrier according to Claim 1 for use in inhibiting the growth of a brain tumor cell, wherein the targeted carrier contains a microRNA or a drug, the microRNA includes miRNA-181, and the drug includes Temodal.

15. The targeted carrier for use according to Claim 14, wherein the brain tumor cell is glioblastoma multiforme (GBM).

16. A pharmaceutical composition comprising the targeted carrier with the property of crossing the blood-brain barrier according to Claim 1.

17. A use of the pharmaceutical composition according to Claim 16 for crossing the blood-brain barrier.

18. A use of a targeted carrier for crossing the blood-brain barrier, wherein the drug carrier comprises an extracellular vesicle, which has a human leukocyte antigen-G antibody bound to its surface.

19. The use according to Claim 18, wherein a transmembrane protein of the extracellular vesicle includes a single-domain antibody fragment bound to the human leukocyte antigen-G antibody.

20. The use according to Claim 18 to 19, wherein the transmembrane protein is selected from the group consisting of CD68, CD81, and CD9, and the single-domain antibody fragment is a variable domain of heavy chain of heavy-chain antibody (VHH) or a single-chain variable fragment (scFv).
